# EUROPEAN PATENT APPLICATION

(11) **EP 4 393 445 A1**
(43) Date of publication of application: **03.07.2024**
(21) Application number: 22216791.8
(22) Date of filing: 27.12.2022
(51) Int. Cl.: A61B 90/11, A61B 17/17, A61B 17/72, A61B 90/14, A61B 90/50, A61B 90/57, A61F 5/01

(54) **ADJUSTING TOOL FOR SUPPORT DEVICES AND SUCH SUPPORT DEVICES**

(71) Applicant: HemiTec Finland Oy, 21290 Rusko (FI)
(72) Inventor: Hyttinen, Mika, 21290 Rusko (FI)
(74) Representative: Boco IP Oy Ab

(57) **Abstract**

The adjusting tool of the invention is intended to be used in a support device. The adjusting tool comprises a first adjustment shaft (34), a second adjustment shaft (35), and a third adjustment shaft (41), and a beam (33) that can be adjusted for rotational movement in at least one direction by means of at least one of said adjustment shafts (34, 35, and/or 41). The first adjustment shaft (34) is attached to the beam (33), the second adjustment shaft (35) is attached to the first adjustment shaft (34), and the third adjustment shaft (41) is attached to the second adjustment shaft (35). Furthermore, the adjusting tool comprises a pair of attachment arms (36). One attachment arm is laterally movable along the beam on one side of the first adjustment shaft and the other one on the other side of the same. The support device of the invention has components to be positioned and supported. The support device is connected to said adjusting tool that on one hand is coupled to a transversal support arm thereof and on the other hand is coupled to a component thereof to be supported. The support device is e.g. a support device for positioning a patient for surgery, such as for correction of bone dislocations or for intramedullary tibial nailing.

## Description

### TECHNICAL FIELD

The invention relates to an adjusting tool and its use for the mutual positioning of components of a mechanical device, especially of a support device. The invention is also concerned with such support devices.

### BACKGROUND

Work-holding, positioning and support devices are used to create a secure point for a workpiece or for work allowing for support during an operation to achieve increased accuracy and precision. Components in such devices should be securely located in a specific position, location and/or orientation so that the devices could support the work in question. Such support devices exist for very different types of work and are designed thereafter for both position, location and support or either one. Locating components can e.g. ensure the geometrical stability of the support device for being in the correct position and orientation for an operation.

The locating components of a support or positioning device can consist of movable and/or rotating arms and shafts kept together by screws or pins and sometimes also of clamps and surfaces. These parts ensure that the components are positioned correctly, and they remain in the same position throughout the operation. Surfaces, arms and shafts provide support, pins and screws allow for precise location, and e.g. clamps allow for the support device or its components to be removed or its position adjusted.

Surgical positioning is the practice of placing a patient in a particular physical position during surgery. The goal in selecting and adjusting a particular surgical position is to maintain the patient's safety while allowing access to the surgical site. Often a patient must be placed in an unnatural position to gain access to the surgical site.

For example, the lithotomy position is a common position for surgical procedures and medical examinations involving the pelvis and lower abdomen, as well as a common position for childbirth in Western nations. In the Trendelenburg position, the body is lain supine, or flat on the back on a 15-30 degree incline with the feet elevated above the head. The reverse Trendelenburg position, similarly, places the body supine on an incline but with the head now being elevated. Especially, intramedullary tibial nailing can be performed with the patient positioned supine on a radiolucent table or on a fracture.

Different positioning and support devices are used for positioning a patient for surgery. These devices are adjusted personally for each patient as a natural consequence of people of different sizes. Thus, there are components in the support devices that are adjusted with respect to their level and location as well as their mutual distance and position, and they can be extended or shortened in different directions, such as vertical and horizontal or angled directions, along with the required adjustment according to e.g. the length and dimensional proportions of the actual person to be fixed in a position for surgery or the like.

Also, the adjustable components of devices used for surgical positioning include arms, shafts, screws, pins etc. Unique positioning accessories and entire such devices have to be designed to achieve specific positions and to provide patients' safety and support. Each such device has a variety of accompanying equipment and accessories, such as parts used to elevate the legs off the surface of the operating table for gynecology, urology, and orthopedic procedures.

One such orthopedic procedure is intramedullary nailing used for treating tibial fractures, which means inserting a metal nail in the medullary cavity of the tibia.

A tibial fracture is a common injury especially among young and middle-aged adults. A fracture may be caused by a fall, an accident or a strong blow to the leg. The treatment of a tibial fracture generally involves repositioning, that is, positioning the ends of the bone at the fracture site in place.

Intramedullary nailing is the most common method for treating tibial fractures. The nail is placed in the medullary cavity through a tendon below the patella. Once the nail is in place, screws are inserted through both of its ends to keep the bones in the desired position.

The function of intramedullary nailing of the tibia is to keep the parts of the bone separated by the fracture aligned so that the ends of the bone at the fracture site of the tibia are in line and the tibia will thus ossify into the correct position.

Traditionally, the intramedullary nail is thus inserted inside the bone from the top part of the tibia, which according to studies causes post-procedural pain in the front part of the knee, that is, so-called Anterior Knee Pain symptoms, to a significant number of patients.

Furthermore, in a still commonly used surgical method, the patient's leg to be operated is placed at a more than 90-degree angle against a round support. In addition, forward and downward traction is applied diagonally to the leg in order to align the ends of the damaged tibia. This procedure may cause the nerves at the back of the knee to become pinched and the risk of nerve damage increases. When using a wedge pillow, an assistant, that is another surgeon, is needed to keep the leg in place. The assistant's task in the procedure is to maintain the required traction in the leg so that the ends of the bone are aligned and the leg is not able to move when space for the intramedullary nail is drilled in the bone.

There are two known versions of intramedullary nailing of the tibia. In the first method, there is used a traction table which is designed for said procedure, but which involves many problems. For example, preparing for surgery is time-consuming and imaging is challenging. The patient may suffer nerve damage if the patient is not positioned correctly, and in this method, the surgeon has to work in an ergonomically demanding working position. In the second method, the intramedullary nailing is performed on a standard operating table using a wedge pillow designed for intramedullary nailing of the tibia. The problem with this procedure is that the leg moves continuously and thus another orthopedist or traumatologist is needed to support the leg during the procedure. In addition, this wedge pillow procedure, in which the leg can move a lot, easily causes tissue damage and vascular damage to the calf muscles.

In a commonly used surgical method, a needle is also drilled through the heel bone and attached to the traction device. There are important nerve structures and soft tissues in the heel bone area, which are more susceptible to damage.

Patent publication CN110537964A is cited as prior art, which discloses a device for intramedullary nailing of the tibia, wherein the length of the bone can be adjusted by means of needles passed through the bone.

The object of the present invention is to provide an auxiliary element to be used as an adjusting element in support devices generally to facilitate the adjusting of components in the support devices that are to be adjusted with respect to their level and location as well as their mutual distance and position.

A special object of the present invention is to provide a device which allows for safer and more patient-friendly tibial intramedullary nailing surgery.

### BRIEF DESCRIPTION OF THE INVENTION

The adjusting tool of the invention is intended to be used in a support device. The adjusting tool comprises a first adjustment shaft, a second adjustment shaft, and a third adjustment shaft, and a beam that can be adjusted for rotational movement in at least one direction by means of at least one of said adjustment shafts. The first adjustment shaft is attached to the beam, the second adjustment shaft is attached to the first adjustment shaft, and the third adjustment shaft is attached to the second adjustment shaft. Furthermore, the adjusting tool comprises a pair of attachment arms. One attachment arm is laterally movable along the beam on one side of the first adjustment shaft and the other one on the other side of the same.

The support device of the invention has components to be positioned and supported. The support device has an adjusting tool that on one hand is coupled to a transversal support arm thereof and on the other hand is coupled to a component thereof to be supported. The adjusting tool comprises a first adjustment shaft, a second adjustment shaft, and a third adjustment shaft, and a beam that can be adjusted for rotational movement in at least one direction by means of at least one of said adjustment shafts. The first adjustment shaft is attached to the beam, the second adjustment shaft is attached to the first adjustment shaft, and the third adjustment shaft is attached to the second adjustment shaft. Furthermore, the adjusting tool comprises a pair of attachment arms. One attachment arm is laterally movable along the beam on one side of the first adjustment shaft and the other one on the other side of the same.

Especially, the support device is a support device for positioning a patient for surgery, such as for correction of bone dislocations or for intramedullary tibial nailing.

The support device of the invention for correction of bone dislocations or for intramedullary nailing of the tibia, comprises the frame of a framework which consists of vertical supports and longitudinal and transverse horizontal supports. It furthermore comprises a traction needle to be passed through the tibia, and a guide, which is slidably coupled to the longitudinal horizontal support of the support device. The guide comprises a first finder for adjusting the position of the traction needle in the lateral direction with respect to the joint surface of the upper part of the tibia, and a second finder for adjusting the position of the traction needle in the vertical direction and for passing the traction needle through the tibia in the transverse direction of the tibia. First attachment arms are configured to be attached to one end of the frame and to the ends of the traction needle that has been passed through the tibia in the transverse direction by using the guide. The support device further comprises an adjusting tool coupled to a horizontal support slidably attached between some of the longitudinal supports in the frame at the other end of the frame than the first attachment arms. The adjusting tool comprises a first adjustment shaft, a second adjustment shaft, and a third adjustment shaft, and a beam that can be adjusted for rotational movement in at least one direction by means of at least one of said adjustment shafts. The first adjustment shaft is attached to the beam, the second adjustment shaft is attached to the first adjustment shaft, and the third adjustment shaft is attached to the second adjustment shaft. Furthermore, the adjusting tool comprises a pair of attachment arms. One attachment arm is laterally movable along the beam on one side of the first adjustment shaft and the other one on the other side of the same.

The preferred embodiments of the invention have the characteristics described below which are disclosed in the dependent claims.

The terms positioning and location as used in the context of the present invention includes all kind of displacements, such as axial displacement, circumferential displacement, rotary motion and linear motion to bring the components into a desired position.

In the preferred embodiments, the first adjustment shaft, the second adjustment shaft and the third adjustment shaft are perpendicular to each other.

Said rotational movement includes a first rotation of the beam around its middle point in the horizontal plane. It is in another words a rotation of the beam about a perpendicular axis in the horizontal plane. Said rotational movement can also include a second rotation of the beam around its middle point in the vertical plane. It is in another words a rotation of the beam about a perpendicular axis in the vertical plane. Still further, said rotational movement can include a third rotation of the beam being an angular rotation along a circle arc, defined by the distance between the third adjustment shaft and the beam, the radius of curvature. In another words, there is a circular motion of the beam following a circular path back and forth when the third adjustment shaft is turned back and forth.

The first adjustment shaft, the second adjustment shaft, and the third adjustment shaft can be turned with respect to each other by means of locking crews, in the respective adjustment shaft simultaneously or separately.

There is a first locking screw for the first rotation and a second locking screw for the second rotation. The first locking screw for its part keeps the first adjustment shaft in place but for additional security, there can be a locating screw to be fastened to the second adjustment shaft to further ensure keeping the first adjustment shaft in place. There is a third locking screw for adjustment of the angular rotation. A fourth locking screw goes perpendicularly to the first adjustment shaft to secure it to the beam.

If the locking screws are loosened simultaneously, combined types of rotations are achieved for the adjustment. If for example the first and second locking screws are loosened simultaneously, the beam might move e.g. in some other reference plane than a horizontal or vertical plane. The adjustment can thus be made by means of two or three locking screws at the same time.

The adjustment tool can be used in different types of support devices. In some uses only one or two types of rotation movement of the beam is needed to achieve a useful adjustment of components in the support device.

When the adjustment tool of the invention is used in a support device for positioning a patient for surgery, such as for correction of bone dislocations or for intramedullary tibial nailing, all three types of adjustment, i.e. the rotation adjustment of the first type about its middle point in the horizontal plane, the rotation adjustment of the second type about its middle point in the vertical plane, and the angular rotation performed as a circular motion of the beam, are usually needed for an exact adjustment and even performed preferably simultaneously. However, even in that case, sometimes only one or two types of rotation adjustments might be sufficient, and it is determined case by case, for example depending on the size and dimensional proportions of the patient.

The third adjustment shaft can be a two-part shaft, the parts of which are secured to each other by screws. The third adjustment shaft preferably has the form of a ring for attaching it to a support arm of a support device by surrounding the same. Thanks to the two-part design, the adjustment tool can be detached from the support arm and be replaced thereto.

The supporting attachment arms of the adjustment tool are laterally movable along the beam on both sides of the first adjustment shaft. Fifth locking screws secure the supporting attachment arms to a selected place of the beam.

The lateral movement of the supporting attachment arms takes place along at least one groove in the beam by means of gliding screws that keep the supporting attachment arms in the correct position in the beam and allow them to glide along the at least one groove by means of a ball in the end of the gliding screw.

The supporting attachment arms have means for coupling them to another component of the support device, such as wing screws. The means for coupling the supporting attachment arms to another component of the support device, such as to a traction needle, if it is question about a device for surgery, further comprises a fastening plate through which the wing screw is going.

The adjusting tool can be used in different type of support devices by coupling the third adjustment shaft to a support arm thereof and by coupling the supporting attachment arms to another component of the support device for supporting purposes.

The support device of the invention for correction of bone dislocations or for intramedullary nailing of the tibia is suitable for both these types of surgery.

When the support device is especially meant for intramedullary nailing of the tibia, the adjustment tool is not necessary and therefore it is detachable, and instead second attachment arms can be fastened to the frame and so that they, instead of the support attachment arms can be attached to the ends of the traction needle that has been passed through the tibia in the transverse direction by using the guide. This can take place if for example an adjustment by means of such second attachment arms is easier than that using the adjustment tool.

However, for correction of bone dislocations, it is preferable to use the adjustment tool.

The support device of the invention for correction of bone mislocations or for intramedullary nailing of the tibia comprises first attachment arms for the traction needle of the lower part of the tibia. The first attachment arms are attached to a transverse support slidably attached between some of the longitudinal supports in the frame. The transverse support slidably attached between the longitudinal supports slides along the longitudinal supports by means of a guide carriage, such as a traction carriage.

The longitudinal horizontal supports usually consist of upper supports and lower supports, in which case the lower supports preferably act as the slide rails for the guide carriage.

The device for intramedullary nailing of the tibia also comprises second attachment arms for the traction needle of the upper part of the tibia. The second attachment arms are attached in the frame to a transverse support attached between two vertical supports in a fixed manner, that is, non-movably.

The transverse supports can be turned forwards and backwards, which means that the attachment arms attached to them can be turned to forward and rearward positions.

The guide comprises a first finder attached with attachment means and a second finder attached with attachment means for passing the traction needles through the lower part and upper part of the tibia, respectively.

A vertical height adjustment part is connected to the guide, which vertical height adjustment part is attached at its upper part to the said same attachment means or to a separate attachment element in the guide, and at its lower part to a second attachment means. The first finder and the second finder are in the same guide at different adjustable heights.

All attachment arms, the first ones, the support attachment arms and possible second attachment arms, are attached to the transverse supports by means of rings which encircle them. The rings comprise a threaded hole through which a screw can be tightened to the bottom of a groove to keep the attachment arms in place.

The device according to the invention for intramedullary nailing of the tibia, which can be called a tibial traction and repositioning device, has been developed for "suprapatellar" (i.e. performed under the patella) intramedullary nailing of the tibia in adult patients.

In the tibial traction and repositioning device according to the invention, the knee is placed at an angle of approximately 5-10 degrees, which prevents the operation from causing nerve damage to the patient's nerves at the back of the knee, in the "crook", because no pressure is exerted on the back of the leg. The device comprises needle attachment arms for the needles at the upper and lower parts of the tibia. The attachment arms of the needle at the lower part of the tibia are on slide rails and thus by pulling the attachment mechanism of the lower part, the ends of the tibia can be aligned. The attachment arms of the needle at the upper part of the tibia do not move in the longitudinal direction of the device.

The adjustment tool is designed so that it, thanks to its versatile functions, facilitates tibial corrective osteotomy, i.e. corrective surgery of the tibia, as well as the connection of an external anchor device to the tibia, in other word an external fixation device to keep fractured bones stabilized and in alignment. Such a fixation device can be adjusted externally to ensure that the bones remain in an optimal position during surgery and/or healing. The adjustment tool of the invention can be used as a further improvement by connecting it to the distal end, i.e. ankle end, of the tibial traction and repositioning device of the invention or the device for correcting bone dislocations to a horizontal support.

The adjustment tool of the invention gives an essential improvement for achieving accurate result in osteotomy surgery, wherein correction of dislocations in the middle part of the tibia or dislocations that do not extend all the way to the joint surfaces are performed. Correct angles and correction of rotation failures can be achieved by using the adjustment element of the invention in accordance with an action plan made after magnetic resonance imaging or native X-ray examination.

The essential functions of the adjustment tool are that the support attachment arms move laterally and simultaneously along the beam to which they are fastened, and the beam can be rotated in different directions. Addition, the right length of the bone can be achieved by using the guide carriage sliding long the lower supports. All these adjustments can be locked for the surgery in a desired position and the surgery ca be performed by a single surgeon.

The adjustment tool of the invention can also be used in devices for correction of congenital bone dislocations. Even for High Tibia Osteotomy (HTO) operations of the upper part of tibia HTO, the device of the invention provides a considerable improvement compared to conventional methods for intramedullary nailing of the tibia. By using the device of the invention, a correction of the bone dislocation is considerably facilitated since the foot can be kept stable in place for the surgery and the adjustment tool can be gradually adjusted in accordance with a grade scale, from which it can directly be seen how much the distal end of the tibia has to be turned in order to place the tibia in the correct position.

The adjustment tool of the invention can also be used in devices for performing rotation osteotomy, wherein an abnormality in the rotation direction of the tibia is corrected. In rotation osteotomy, the tibia is cut off completely and the right position is achieved by rotating the tibia in the right direction. In such surgery, the adjustment tool of the invention provides a big advantage, since the tibia will not be shortened when cut off, since it is fastened to the device at tis proximal and distal end. A very accurate rotation failure can be achieved by using the adjustment tool of the invention in the device as the distal axis of the adjustment tool rotates thirty degrees.

In an embodiment of the invention, the tibia is attached to the tibial traction and repositioning device by means of thin needles. The needles are drilled through the bone from the side of the tibia, from the upper and lower parts of the fractured tibia. The advantage of the novel device is that the needles are drilled into already damaged bone, thus sparing intact bones, nerves and tissues from unnecessary damage.

In one embodiment of the medico-technical device according to the invention allows for safer and more cost-efficient intramedullary nailing surgery of the tibia. In conventional methods, the intramedullary nailing of the tibia on a pillow requires an additional surgeon for the procedure in order to provide sufficient traction in the leg and to keep the leg in place during the procedure.

The invention is described in greater detail in the following by means of an embodiment and a related figure as well as the use of the embodiment.

### FIGURES

Figure 1 shows the adjusting element of the invention
Figure 2 is an example of a support device of the invention in the form of device of the invention for correcting bone dislocations or for intramedullary nailing of the tibia
Figure 3 is a detailed view of a part of figure 2

### DETAILED DESCRIPTION

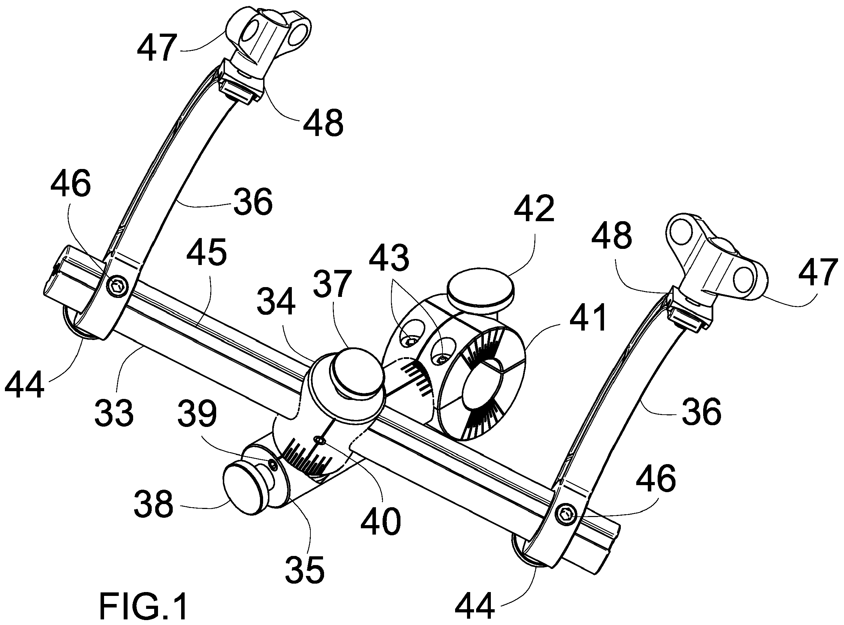

Figure 1 shows the adjustment tool of the invention. The adjustment tool comprises a beam 33 that can be adjusted for rotation of different types or a combination of those.

A first adjustment shaft 34 is coupled to the beam 33 for rotation of the beam about its middle point in the horizontal plane, i.e. about a perpendicular axis in the horizontal plane. This rotation is here called first rotation. The beam 33 goes through the first adjustment shaft 34. There is a first locking screw 37 on top of the first adjustment shaft 34 for the adjustment of the first rotation by loosening of the first locking screw 37. The shaft 34 can be turned in both directions to left and right and by using a grade scale marked on it.

A second adjustment shaft 35 is coupled to the first adjustment shaft 34 for rotating the beam 33 about a perpendicular axis in the vertical plane, i.e. around its middle point in the vertical plane. This rotation is here called second rotation. A second locking screw 38 in the end of the second adjustment shaft 35 can be loosened for adjustment of the second rotation of the beam 33. Also shaft 35 can be turned in both directions to left and right and by using a grade scale marked on it as a help.

A locating screw 39 to be fastened to the second adjustment shaft 35 can be used as an additional security to keep the first adjustment shaft 34 in place.

A fourth locking screw 40 goes perpendicularly to the first adjustment shaft 34 to secure it to the axis 33.

When the screws 39 and 40 are on eachother the axes are not turned with respect to eachother. In figure 1, it can be seen that the adjustment shaft 34 has been turned about 30 degrees to the left with respect to the adjustment shaft 35.

A third adjustment shaft 41 is used for turning the second adjustment shaft 35 back and forth (or up and down depending on perspective) and therethrough the entire adjustment tool. This causes an angular rotation of the beam about the third adjustment axis 41 being a circular motion along a circular arc. There is a third locking screw 42 for adjustment of the turning of the third adjustment axis 41 i.e. to perform the angular rotation. Also, shaft 41 can be turned in both directions to left and right and by using a grade scale marked on it as a help.

The third adjustment shaft 41 is a two-part shaft, the parts of which are secured to each other by screws 43. The third adjustment shaft 41 has the form of a ring for attaching it to a transverse support arm of a support device by surrounding the same for installing. The third locking screw 42 also attaches the third adjustment shaft 41 to a support arm of a support device of the invention.

The first adjustment shaft 34, the second adjustment shaft 35 and the third adjustment shaft 41 are perpendicular to each other. Figure 1 is seen as a perspective view from the above. The third adjustment shaft 41 can be turned so that the circular motion of the beam 33 takes place and it is perpendicular to the second adjustment shaft 35 as well as to the first adjustment shaft 34.

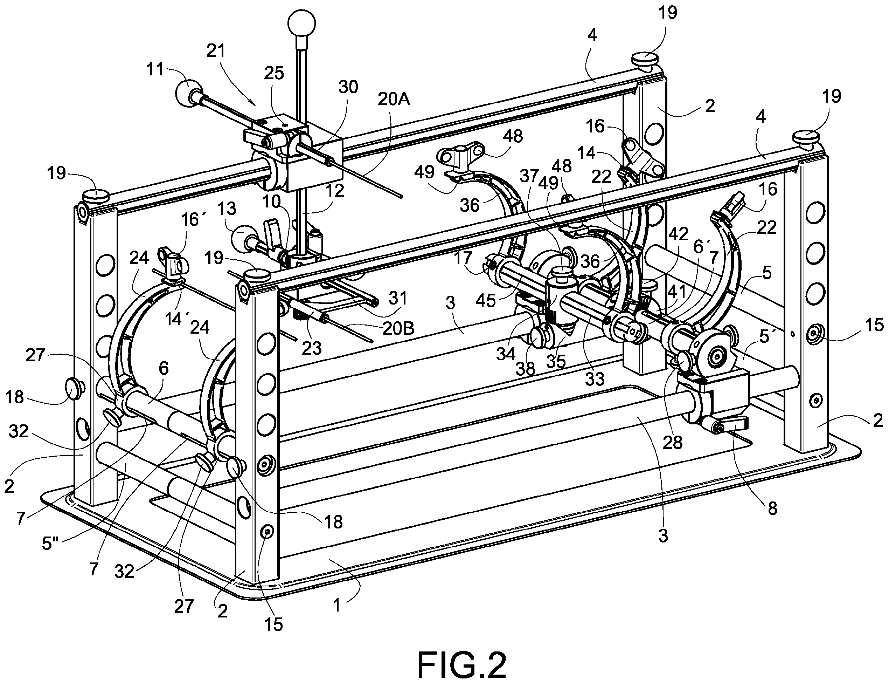

There is a pair of support attachment arms 36 so that there is a support attachment arm 36 laterally movable along the beam 33 on each side of the first adjustment shaft 34. In figure 2, the lower ends of the support attachment arms 36 are configured to surround the axis 33. Alternatively, there could be in the ends of the arms or slots for inside movement. Fifth locking screws 44 secure the attachment arms 36 to a selected place of the beam 33.

The lateral movement of the attachment arms 36 takes place along at least one groove 45 in the beam 33 by means of gliding screws 46 that keep the support attachment arms 36 in the correct position in the beam 33 and allow them to slide along the at least one groove 45 by means of a ball in the end of the gliding screw 46.

The attachment arms 36 have means, such as wing screws 47, for coupling them to another component of a support device, such as to a traction needle of a device for correction of bone dislocations or for intramedullary nailing of the tibia. The means for coupling the support attachment arms 36 to another component of the support device further comprises a fastening plate 48 through which the wing screw 47 is going.

Figure 2 is an example of a support device of the invention in the form of a traction and repositioning device of the invention for intramedullary nailing of the tibia. The device is for correction of bone dislocations or for tibial traction and repositioning for intramedullary nailing of the tibia, wherein an intramedullary nail is inserted in the tibia.

A tibial fracture inflicted on a patient due to a trauma is treated by means of an intramedullary nail inserted in the tibia and left there if repairing the tibia so requires.

This is the case if the bone is fractured into two or more parts and the fractures do not extend to the joint surface.

The patient (not shown) lies on their back on the operating table (not shown) throughout the operation. The patient is anaesthetised for the procedure. The patient is then covered with sterile covering, leaving the leg to be operated exposed.

The device according to the invention has a rectangular base 1. The base 1 is a bottom plate, which is a rectangular plate open in the centre to allow imaging so that there is no metal in the imaging area when imaging from above with respect to the leg. The purpose of the base 1, that is, the bottom plate, is to stabilise the device and to prevent the device from pressing into the soft mattrasses of the operating table. The vertical supports 2 can be attached to the base 1.

Two upper supports 4 are attached to the totally four vertical supports 2, in each corner of the base 1, by means of knurled-head screws 19, approximately at the height of the tibia when the knee is elevated to an angle of approximately 10 degrees. The two upper supports 4 are attached on both sides of which base 1 by extending over its longitudinal sides. In addition, two lower supports 3 extending over longitudinal sides of the base 1 are coupled approximately to the level of the ankle.

Transverse supports 5, 5', 5" and 6, which are attached to the vertical supports 2, for example, by means of screws 15, approximately to the level of the ankle, are coupled on the other two sides of the base 1, by extending over its short sides. The device further comprises a transverse support 6' between the lower supports 3, which moves on a guide carriage 8, such as a traction carriage, along the lower supports 3. The lower supports 3 have thus been constructed to function as slide rails for the guide carriage 8 and support the device.

The vertical supports 2, the upper supports 4, the lower supports 3 and the transverse supports 5, 5', 5", 6 and 6' may be, for example, guides, pipes or rods or corresponding supports or shafts.

In Figure 2, the lowest transverse supports 5' and 5" are on a somewhat lower level than the lower supports 3, and transverse support 5 and transverse support 6 are on a somewhat higher level than the lower supports 3. Transverse support 6 is drawn discontinuously in Figure 2 for illustrative purposes to make it easier to see how first attachment arms 24 are attached to it, which will be explained later in the text. Transverse support 6 is a control shaft, the function of which will be explained below. In Figure 2, the transverse supports 5, 5', 5" and 6 are attached to the vertical supports 2 by means of fastening screws 15 or by another method of attachment. The purpose of the holes (no reference numeral) in the vertical supports 2 is to lighten because the vertical supports 2 are heavy. The transverse supports 5, 5', 5" and 6 are in their fixed positions and the transverse support 6' moves along the lower supports 3 as mentioned above.

The device according to the invention is placed under the leg to be operated in such a way that the patient lies on his back and the leg to be operated on the device is towards shaft 5.

After this, the leg to be operated must still be placed accurately or more accurately in position on the device to stabilise the bone with the fracture.

For stabilisation, so-called traction needles are drilled through the tibia laterally and both in the upper part of the tibia, in the vicinity of the patella, at a point below it, and in the lower part of the tibia, at the ankle. Drilling the traction needles through the tibia takes place by means of a guide attached to an upper support 4. The guide 21 moves along another upper support 4.

The traction needles will be removed before the surgical wound is closed after the operation. Before drilling the traction needles, it, however, has to be ensured that the guide 21 for drilling a traction needle is in the correct position with respect to the bone in both the vertical and lateral directions. The position of the guide 21 is checked with an X-ray machine and adjusted if necessary.

The guide 21 moves unobstructed in the upper support 4 from the ankle to the knee and comprises separate transverse arms 11, 13 at different heights which function as finders for positioning the traction needles. These transverse arms 11, 13 are directionally controlled and not intended to rotate, and thus their shape is preferably other than round, for example, a square, thus locking them better to the sleeve in the lateral direction to prevent rotation.

### Positioning the first traction needle

The guide 21 comprises a first sleeve 25, through which a first transverse arm 11 passes, transversely to the upper support 4, at the end of which transverse arm 11, there is shown a traction needle 20 in position A, at which its lateral position is adjusted. The traction needle 20 is marked as 20A when it is in position A in the sleeve 30 at the end of the transverse arm 11. The transverse arm 11 functions as the upper finder for the upcoming mounting of the traction needle 20. The purpose of the transverse arm 11, that is, of the upper finder, is to adjust the lateral position of the guide 21, which is done by X-raying from above. The actual drilling of the traction needle 20 through the tibia does not yet take place in position A.

The guide 21 comprises a second sleeve (not visible being on the other side of the guide 21), through which passes a height adjustment part 12, the second sleeve being in the upper part of the height adjustment part 12. A bar or the like can be used as a height adjustment part 12. In the lower part of the height adjustment part 12, there is a third sleeve 10 through which the height adjustment part 12 of the guide 21 passes. The height adjustment part 12 thus combines the lower finder with the upper finder (i.e. transverse arms 11 and 13).

A second transverse arm 13 passes through sleeve 10, transversely to the upper support 4, to which second transverse arm 13, the traction needle 20 is now attached by means of a connecting piece 31, which comprises a sleeve 23. In other words, the traction needle is transferred from transverse arm 11 to transverse arm 13. Transverse arm 13 functions as a lower finder for the same traction needle 20 which was previously used to check at which point the traction needle 20 was with respect to the joint surface of the upper part of the tibia, that is, its position in the lateral direction was checked and it was then transferred into the sleeve 23 of transverse arm 13.

Thus, after the adjustment of the lateral direction of the guide 21, this same traction needle 20 is drilled through the sleeve 23 of the lower finder and further through either the upper part or lower part of the tibia once it has first been ensured by an X-ray that the traction needle 20 is at the correct height by X-raying from the lateral direction.

It is further emphasised that this traction needle 20 is transferred from the upper finder to the lower finder once the leg has first been X-rayed from above and it has been ensured that the traction needle 20 will not penetrate the joint and remains below the joint surface. The upper finder and the lower finder are positioned on top of one another when viewed from above, and the said finders are aligned on top of one another when the device is viewed from the lateral direction.

When the position of the guide 21 has been ensured and correctly located, the drilling of the traction needle 20 through the bone takes place. This is usually done so that an equal part of it is exposed on each side of the leg. For drilling through the bone, the traction needle 20 is attached to a separate hand drill (not shown) before it is drilled through the sleeve 23 into the tibia, that is, once the guide 21 and thereby the sleeve 23 have been set to the correct height with respect to the tibia by using the height adjustment part 12. X-raying from above shows how close the needle is to the joint surface.

The traction needle 20 is marked as 20B when it is in position B drilled into the bone. Position B thus describes traction needle 20B in its position B drilled inside the bone. The order of positioning the needles 20 is not relevant, but it is more common to position the traction needle of the upper part of the tibia first. In this embodiment it was, therefore, assumed that the traction needle 20 is first drilled to the upper part of the tibia.

The traction needle 20 in the upper leg is attached at both of its ends, for example, by means of winged screws 16' and clamps 14' to the first attachment arms 24 intended for them, with the leg at a small, approximately 1 0-degree angle. The first attachment arms 24 are in turn attached by means of rings 27 to a fixed transverse support 6, which thus passes through the ring 27. Alternatively, the lower end of the first attachment arm 24 could be configured, e.g. bent, around the transverse support 6.

The first attachment arms 24 are attached to a transverse support 6 by means of rings 27 to enable them to turn forwards and backwards. The attachment arms 24 turn along with the transverse support 6 when the screw 18 is loose. The first attachment arms 24 can thus be turned into a rear position out of the way when the patient is fitted with a traction needle.

The first attachment arms 24 also move slightly laterally along the transverse support 6 when installed knurled-head screws 32 are loose. On the outer surface of the transverse support 6, at the knee end, that is, at the end of the upper leg, there is a groove 7 which remains on the inner side or the outer side of the device.

The purpose of the groove 7 is to enable locking the first attachment arms 24 with a tightening screw, such as a knurled-head screw 32, to a position in which they are sufficiently close to the skin for attaching the traction needle 20 to the first attachment arms 24. In other words, the attachment arms 24 are first placed close to the skin and then locked with knurled-head screws 32 in the transverse groove 7 on the outer surface of the transverse support 6. The groove 7 prevents the first attachment arms 24 from rotating because a part of the tightening screw is at the bottom of the groove 7. If there was no groove 7, tightening the first attachment arms 24 against the rounded surface of the transverse support 6 might lead to the first attachment arms 24 being able to rotate.

### Positioning the second traction needle

Positioning the second traction needle, in this embodiment to the lower part of the tibia, is carried out with the same guide 21 as the first traction needle 20 was positioned to the upper part of the tibia.

When the position of the second traction needle in the longitudinal direction has been adjusted with the upper finder 11, as was done with the first traction needle, the second traction needle is positioned to the end of the transverse arm 13 functioning as the lower finder by means of a connecting piece 31 and the sleeve 23 therein.

Drilling the second traction needle through the tibia in its bottom part at the ankle takes place in the same way as the drilling of the first traction needle 20 to the upper part of the tibia.

Since it is assumed that Figure 2 shows the first traction needle 20, which is drilled into the upper part of the tibia, the second traction needle has not been given its own reference numeral, but Figure 2 is exactly equivalent when the question is of positioning the second traction needle, with the exception that the first traction needle 20 is replaced by the second traction needle.

The traction needle of the lower leg is attached at both its upper ends, for example, by means of wing screws 47 and clamps or fastening plates 48 to support attachment arms 36 intended for them. The support attachment arms 36 are part of the adjusting element of the invention, which is presented in more detail in figure 1.

The adjusting tool is attached to the moving transverse support 6', with the leg at a small angle of approximately 10 degrees.

The adjusting tool is detachable and can be removed from the device and instead, the traction needle of the lower leg can be attached to second attachment arms 22 instead of attaching the traction needle to the support attaching arms 36.

In that case, the traction needle of the lower leg is attached at both its ends, for example, by means of wing screws 16 and clamps 14 to second attachment arms 22 intended for them which are attached to the moving transverse support 6', with the leg at a small angle of approximately 10 degrees.

The second attachment arms 22 are attached to transverse support 6' by means of rings 27 to enable them to turn forwards and backwards. The second attachment arms 22 turn along with the transverse support 6' when the screw 28 is loose. The second attachment arms 22 can thus be turned into a rear position out of the way when the patient is fitted with a traction needle. The movement of the second attachment arms 22 forwards and backwards can thus be adjusted with a screw 17, by means of which the transverse support 6' can be rotated.

The second attachment arms 22 also move somewhat sideways along the transverse support 6' when knurled-head screws 32 are loose.

To position the traction needle, that is, the traction needle of the lower leg, the second attachment arms 22 are first placed close to the skin and then locked with knurled-head screws 32 to the transverse support 6' moving along the lower support 3, in a groove 7 on the outer surface of the transverse support 6'. At the end of the ankle, that is, at the lower leg end, the groove remains on the outer side outwards from the device.

Once the traction needle is positioned, the screw 17 is turned to the front position and locked with a knurled-head screw 32.

Some additional points of emphasis are disclosed below.

Generally speaking, if the knurled-head screw 32 is loose, the first and second attachment arms 22 or 24 and the support attachment arms 36 can be moved laterally on shaft 6 or 6' at the same time when the traction needle 20 is already attached to the first and second attachment arms 22 or 24 or to the first attachment arms 24 and the support attachment arms 36. The rods 6, 6' and the first and second attachment arms 22, 24 and the support attachment arms 36 may not under any circumstances move forwards or backwards after the traction needles 20 have been attached. If the knurled-head screw 32 was so loose that the screw was no longer in the groove 7, then the first and second attachment arms 22, 24 and the support attachment arms 36 could rotate freely about the shaft 6, 6' but this must not happen.

The purpose of the topmost long upper supports 4 of the device in the longitudinal direction is to stabilise the device. They can also be used to support the leg, for example, by using an X-ray drape during the actual intramedullary nailing procedure or in positioning the traction needles at the very beginning of the procedure. Supporting with an X-ray drape means, for example, that the drape can be placed so that it is attached at both ends of the drape to the upper supports 4 with drape forceps. One end of the drape in one upper support 4 and the other end in the other. In this way, the leg can be placed on the drape/drapes when, for example, placing the traction needles in position, and thus the leg does not need to be supported.

The upper supports 4 are usually on purpose slightly higher than the attachment arms of the traction needles when they are in the front position. This is to keep the upper supports 4 out of the way of the X-raying when imaging from the lateral direction with respect to the leg.

The guide 21 used at the beginning of the procedure is also attached to the upper supports 4. The guide 21 slides on the upper support 4 unobstructed from the knee to the ankle. The guide 21 is always attached to the outermost long upper support 4 with respect to the leg to be operated. A traction needle is drilled through the tibia through the sleeve of the guide 21 which is why it is always positioned in the outermost upper support 4. The guide 21 has two functions: the upper finder 11 and the lower finder 13. The upper finder 11 comprises a transverse rod 30, which is hollow at the end of the rod to allow a traction needle to be placed at the end of the rod. The upper finder 11 shows with an X-ray device, with the help of a traction needle, how far the traction needle is from the joint surface, that is, the X-ray is taken from above with respect to the leg. The lower finder 13 is used to view the correct height for the traction needle with the X-ray device from the lateral direction with respect to the leg. The lower finder 13 also comprises a guide sleeve 23, through which the same traction needle which was first used in the upper finder is drilled through the bone. The lower finder also comprises a height adjustment part 12, for example a vertical rod, by means of which the height of the sleeve with respect to the bone can be adjusted. These two functions, the upper and lower finder, are obviously aligned with respect to each other when the entity is viewed from above.

The upper supports 4 can be detached from the device when the intramedullary nail has been positioned in the tibia, but this is not necessary. The device can be constructed so that the upper supports 4 are not in the way when the fastening screws are placed on the nail.

In addition to stabilising the device, the long lower supports 3 also act as slide rails for the guide carriage 8, or traction carriage, at the ankle end. The carriage allows the right amount of traction to be exerted on the leg for aligning the ends of the bone and to lock the guide carriage 8 to the lower support 3 so that it cannot move by itself.

The lower transverse supports 5' and 5" are so-called fixed transverse supports and they can be removed, if necessary, but their purpose is to stabilise the device. The total of the two first attachment arms 24 of the traction needles are attached to the upper transverse support 6. There is also a milled grooving at both ends of the transverse support 6 of the first attachment arms 24, which means that the transverse support 6 can be rotated into the rear position when the traction needles are being positioned in the patient with a finder 13. Once the traction needle has been positioned, the transverse support 6 is rotated into the front position and locked into it with a knurled-head screw 18 to prevent the first attachment arms 24 from swinging.

The guide carriage 8 (i.e. traction carriage) moves unobstructed on the lower supports 3 from the ankle end to the knee end. The guide carriage 8 comprises a bushing/sleeve (no reference numeral) for each of the lower supports 3, through which the lower supports 3 pass, and the sleeve has some kind of a locking screw (no reference numeral) with which the guide carriage 8 can be locked to the desired position on the lower support 3. On top of the lower sleeves, through which the lower support 3 passes, there is a separate attachment mechanism for the transverse supports of the attachment arms of the traction needles, which mechanism is attached to the lower sleeves. The first attachment arms 24 of the traction needles of the ankle function in the same way as in attaching the knee, that is, the first attachment arms 24 can be locked into the front position and the transverse support 6 has a groove 7 and the first attachment arms 24 of the traction needle can thus be moved in the lateral direction and be locked into the desired position in the groove 7 with a knurled-head screw.

The upper supports 4 are usually at an approximately 5-7 cm higher level than the traction needles which are drilled through the tibia and attached to the first and second attachment arms 22, 24 or to the first attachment arms 24 and the support attachment arms 36 intended for the traction needles. The size of the leg is, therefore, irrelevant because the thickness of the human tibia is relatively constant in adult patients and the needle is usually drilled into the centre of the bone. The upper support 4 is, therefore, never in the way of imaging. The height of the ankle is on the same level as the second attachment arms 22 or the support attachment arms 36.

The transverse support 6' is made to move along the lower support 3 to allow the leg to be placed in traction and it is placed in position on the lower support 3 where the leg is in correct traction. For this purpose, the second attachment arms 22 or the support attachment arms 36 for the traction needle on the lower part of the tibia are on slide rails by means of the transverse support 6' and thus by pulling the attachment mechanism of the lower part, the ends of the tibia can be aligned.

The leg is placed in traction in the longitudinal direction and repositioned in the lateral direction by aligning the ends of the bone in the fracture site, for example, by using an X-ray dressing. An X-ray dressing is a lint-free cloth with a small piece of metal thread inside. The purpose of the metal thread in the cloth is (if these cloths are used in operations where a cloth is placed inside the body of a person), to enable finding the cloth by X-ray imaging thanks to the metal thread, because otherwise finding a cloth covered in blood is in practice impossible. The cloth is approximately 40 cm long and, when folded open, approximately 30 cm wide. The cloths can also be used to help in attaching the traction needles by placing the cloth transversely on the upper supports 4 so that the leg rests supported on the cloths. The leg will then not have to be supported when positioning the needles and the leg stays in place better.

There is a thread in the hole in the rings 27 of the transverse support 6' and the first and second attachment arms 22, 24 and the support attachment arms 36 can be held in place with a knurled-head screw 32 when the knurled-head screw 32 is tightened to the bottom of the groove, and when it is loosened, the first and second attachment arms 22, 24 or the first attachment arms 24 and the support attachment arms 36 can again be moved simultaneously. It also prevents the attachment arms 22, 24, 36 from turning downwards due to the weight of the leg because the screw 32 is in the groove and not against a rounded surface. Similarly, the rings 27 in transverse support 6 comprise knurled-head screws not shown in Figure 1, which are used for tightening in a corresponding manner.

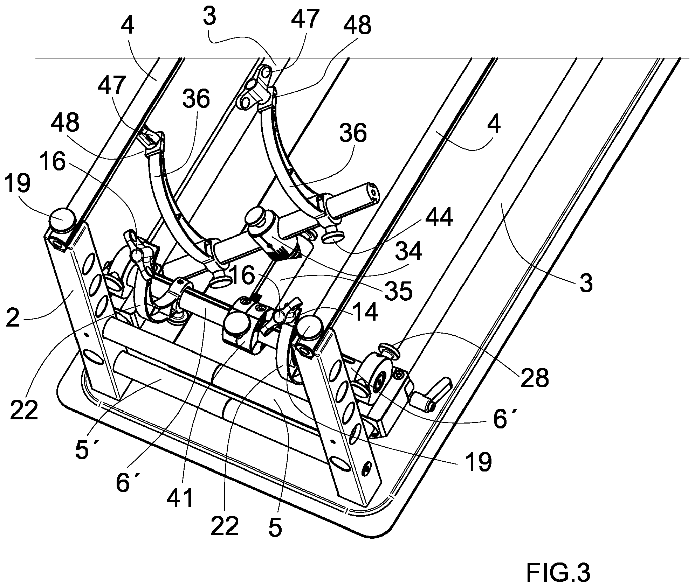

Figure 3 is a detailed view of a part of figure 2.

Once the traction needles 20 are in place, the actual intramedullary nailing is carried out:
- Next the surgeon makes an incision above the patella, through which the instruments used for the incision are inserted from under the patella inside the tibia.
- A tissue protector is first placed under the patella, which tissue protector prevents the instruments from damaging the tissues and other important areas which are not relevant to the operation itself. All instruments required in the procedure, except the intramedullary nail, are inserted in the tibia through the tissue protector.
- Next an entry opening is drilled in the bone with a slightly larger drill bit to a depth of approximately 4 cm in the longitudinal direction of the bone.
- A guide wire which reaches all the way down to the ankle is then inserted in the bone. The guide wire has a thickness of approximately 2 mm-3 mm. It is used because the drill which is used to drill bone marrow away from obstructing the nail is hollow and the guide wire runs inside the drill bit to prevent the bit from penetrating the tissues at the fracture site and causing damage to the tissues.
- After this, the bone marrow is gradually drilled, "reamed", away from inside the bone by changing the drill bit to a 1.5 mm larger one after each drilling until there is enough space for the intramedullary nail.
- The intramedullary nail is then inserted in the bone with the help of the guide wire
- When the intramedullary nail is inside the bone, the guide wire is removed and the upper horizontal supports 4 are optionally removed from the sides of the device.
- The intramedullary nail is now fixed inside the patient with screws. The upper supports 4 are then optionally removed, after which the leg can be detached from the first and second attachment arms 22, 24 or the support attachment arms 36 for the traction needles and the needle is cut from one side close to the skin and pulled out of the patient with flat nose pliers.
- After these procedures, the incised wounds are closed and covered with wound dressings.

## Claims

1. Adjusting tool for a support device, the adjusting tool comprising a first adjustment shaft (34), a second adjustment shaft (35), and a third adjustment shaft (41), and a beam (33) that can be adjusted for rotational movement in at least one direction by means of at least one of said adjustment shafts (34, 35, and/or 41),
the first adjustment shaft (34) being attached to the beam (33),
the second adjustment shaft (35) being attached to the first adjustment shaft (34), and the third adjustment shaft (41) being attached to the second adjustment shaft (34), the adjusting tool further comprising
a pair of attachment arms (36), wherein there is an attachment arm (36) laterally movable along the beam (33) on both sides of the first adjustment shaft (34).

2. Adjusting tool of claim 1, wherein the first adjustment shaft (34), the second adjustment shaft (35), and the third adjustment shaft (41) are perpendicular to each other.

3. Adjusting tool of claim 1 or 2, wherein the first adjustment shaft (34), the second adjustment shaft (35), and the third adjustment shaft (41) can be turned with respect to each other by means of locking crews (37, 38, and 42), in the respective adjustment shaft (34, 35, and 41) simultaneously or separately.

4. Adjusting tool of any of claims 1 - 3, wherein said rotational movement includes a first rotation of the beam (33) about its middle point in the horizontal plane, whereby there is a first locking screw (37) in the first adjustment shaft (34) for the first rotation adjustment.

5. Adjusting tool of any of claims 1 - 4, wherein said rotational movement includes a second rotation of the beam (33) about its middle point in the vertical plane, whereby there is a second locking screw (38) in the second adjustment shaft (35) for the second rotation adjustment.

6. Adjusting tool of any of claims 1 - 5, wherein said rotational movement includes a third rotation of the beam (33) being an angular rotation along a circle arc, defined by the distance between the third adjustment shaft (35) and the beam (33), whereby there is a third locking screw (42) in the third adjustment shaft (35) for the third rotation adjustment.

7. Adjusting tool of any of claims 1 - 6, further comprising a locating screw (39) to be fastened to the second adjustment shaft (35) to keep the first adjustment shaft (34) in place as an additional security.

8. Adjusting tool of any of claims 1 - 7, further comprising a fourth locking screw (40) going perpendicularly through the first adjustment shaft (34) to secure it to the beam (33).

9. Adjusting tool of any of claims 1 - 8, wherein the third adjustment shaft (41) is a two-part shaft, the parts of which are secured to each other by screws (43).

10. Adjusting tool of any of claims 1 - 9, wherein the attachment arms (36) are laterally movable along the beam (33) on both sides of the first adjustment shaft (34), and fifth locking screws (44) for securing the attachment arms (36) to a selected place on the beam (33).

11. Adjusting tool of any of claims 1 - 10, wherein the lateral movement of the attachment arms (36) takes place along at least one groove (45) in the beam (33) by means of gliding screws (46) that keep the attachment arms (36) in the correct position in the beam (33) and allow them to glide along the at least one groove (45) by means of a ball in the end of a gliding screw (46).

12. Adjusting tool of any of claims 1 - 11, wherein the third adjustment shaft (41) has the form of a ring for attaching it to a support arm of a support device by surrounding the same.

13. Adjusting tool of any of claims 1 - 12, wherein the attachment arms (36) have means for coupling them to another component of the support device, such as wing screws (47).

14. Adjusting tool of claim 13, wherein the means for coupling the attachment arms (36) to another component of the support device further comprises a fastening plate (48) through which the wing screw (47) is going.

15. A support device with components to be positioned and supported, the support device having an adjusting tool of any of claims 1 - 14 on one hand coupled to a transversal support arm thereof and on the other hand coupled to a component thereof to be supported, the adjusting tool comprising a first adjustment shaft (34), a second adjustment shaft (35), and a third adjustment shaft (41), and a beam (33) that can be adjusted for rotational movement in at least one direction by means of at least one of said adjustment shafts (34, 35, and/or 41),
the first adjustment shaft (34) being attached to the beam (33),
the second adjustment shaft (35) being attached to the first adjustment shaft (34), and the third adjustment shaft (41) being attached to the second adjustment shaft (34), the adjusting tool further comprising
a pair of attachment arms (36), wherein there is an attachment arm (36) laterally movable along the beam (33) on both sides of the first adjustment shaft (34).

16. The support device of claim 15, wherein the support device is a support device for positioning a patient for surgery, such as for correction of bone dislocations or for intramedullary tibial nailing.

17. The support device of claim 16 for correction of bone dislocations or for intramedullary nailing of the tibia, comprising
the frame of a framework which consists of vertical supports (2) and longitudinal (3, 4) and transverse horizontal supports (5, 5', 5", 6, 6'),
a traction needle (20) to be passed through the tibia,
a guide (21) which is slidably coupled to the longitudinal horizontal support (4) of the support device and which guide (21) comprises
a first finder (11) for adjusting the position of the traction needle (20) in the lateral direction with respect to the joint surface of the upper part of the tibia, and
a second finder (13) for adjusting the position of the traction needle (20) in the vertical direction and for passing the traction needle (20) through the tibia in the transverse direction of the tibia, and
first attachment arms (24) are configured to be attached to one end of the frame and to the ends of the traction needle (20) that has been passed through the tibia in the transverse direction by using the guide (21), **characterized by** further comprising
an adjusting tool of any of claims 1 - 14 coupled to a horizontal support (6') slidably attached between some of the longitudinal supports in the frame at the other end of the frame than the first attachment arms (24), which adjusting tool comprises a first adjustment shaft (34), a second adjustment shaft (35), and a third adjustment shaft (41), and a
beam (33) that can be adjusted for rotational movement in at least one direction by means of at least one of said adjustment shafts (34, 35, and/or 41),
the first adjustment shaft (34) being attached to the beam (33),
the second adjustment shaft (35) being attached to the first adjustment shaft (34), and the third adjustment shaft (41) being attached to the second adjustment shaft (34), the adjusting tool further comprising
a pair of attachment arms (36), wherein there is an attachment arm (36) laterally movable along the beam (33) on both sides of the first adjustment shaft (34).

18. A device according to claim 17, wherein when the device is to be used as a device for intramedullary nailing of the tibia, it further comprises second attachment arms (22), which are configured to be attached to the frame and so that they, instead of the support attachment arms (36) can be attached to the ends of the traction needle (20) that has been passed through the tibia in the transverse direction by using the guide (21), and wherein the third adjustment shaft (41) is a two-part shaft, the parts of which are secured to each other by screws (43) for allowing an easy detachment of the adjustment element.

19. A device according to claim 17 or 18, wherein the transverse support (6') slidably attached between the longitudinal supports slide along the longitudinal supports by means of a guide carriage (8), and wherein the longitudinal horizontal supports consist of upper supports (4) and lower supports (3), which lower supports (3) act as slide rails for the guide carriage (8).

20. A device according to claim 19, wherein the angular rotation of the beam (33) can be performed by means of the guide carriage (8) via the slidable transverse support (6'), which is fastened to the third adjustment shaft (41).

21. A device according to any of claims 17 - 20, wherein the transverse supports (6', 6) are configured to be turned forwards and backwards, which means that the attachment arms (36, 22 and 24, respectively) attached to them can be turned to forward and rearward positions.

22. A device according to any of the claims 14 - 21, wherein the attachment arms (36, 22, 24) are attached to the transverse supports (6' and 6, respectively) by means of rings ( 27, 27') surrounding them or by having ends that surround the transverse supports (6' and 6, respectively), the rings (27, 27') or ends comprising a threaded hole through which a screw (44, 32) can be tightened to the bottom of a groove to keep the attachment arms (36, 22, 24) in place.
